# DEMANDE DE BREVET EUROPEEN

(11) **EP 2 949 201 A1**
(43) Date de publication de la demande: **02.12.2015**
(21) Numéro de dépôt: 15170079.6
(22) Date de dépôt: 01.06.2015
(51) Int. Cl.: A01G 9/02, A01G 9/00, G01N 31/22, G01N 33/24, A01G 27/00

(54) **ETIQUETTE COMPRENANT UNE ENCRE HYDROCHROME ET RÉCIPIENT COMPORTANT UNE TELLE ÉTIQUETTE**

(30) Priorité: 30.05.2014 FR 1454910; 01.12.2014 FR 1461699
(71) Demandeur: INLAB, 38170 Seyssinet Pariset (FR)
(72) Inventeur: Lagoutte, Sébastien, 38000 Grenoble (FR); Sivera Bourgouin, Caroline, 38360 Engins (FR)
(74) Mandataire: Regimbeau

(57) **Abrégé**

La présente invention se rapporte à une étiquette destinée à être intégrée à la paroi d'un récipient, caractérisée en ce qu'elle comprend :
- une première couche (20) d'un matériau poreux, c'est-à-dire perméable à l'eau ;
- une deuxième couche colorée (21) non soluble dans l'eau recouvrant au moins une partie de la première couche (20) ;
- une troisième couche (22) d'une encre hydrochrome qui recouvre au moins une partie de la deuxième couche colorée, cette encre étant opaque en l'absence d'eau et transparente en présence d'eau.

## Description

La présente invention est relative à une étiquette destinée à revêtir une paroi d'un récipient, notamment par un procédé de surmoulage, ainsi qu'à un tel récipient. Ce récipient peut avoir une forme et un volume quelconques.

Bien que non limitée à cette application, la présente invention est plus particulièrement orientée sur un récipient que l'on va pouvoir utiliser pour la culture de plantes.

Dans ce contexte, on cherche en effet à déterminer de manière simple le taux d'humidité du terreau contenu dans un tel récipient pour savoir si une plante qui y est cultivée nécessite ou non d'être arrosée.

Cette invention fait usage d'une encre hydrochrome qui présente la particularité de changer d'état au contact de l'eau.

Un tel type d'encre est bien connu et certaines sociétés en font usage en l'intégrant dans des gammes de produits.

Ainsi, on connaît une société britannique qui fabrique des parapluies pourvus de motifs décoratifs. Ces derniers passant d'un état non coloré quand le temps est sec à un état coloré lorsque la pluie tombe sur le parapluie.

Cette même société décline ce concept à des vêtements, ainsi qu'à des rideaux de douche qui "prennent des couleurs" lorsqu'ils sont imprégnés d'eau.

Une autre société spécialisée dans les jouets a revêtu la surface d'un tapis avec une encre hydrochrome.

En l'absence d'humidité, la surface du tapis présente un aspect uniforme.

En revanche, lorsqu'un enfant manipule des jouets contenant de l'eau, il fait alors apparaître des dessins colorés à la surface du tapis qui correspondent aux motifs qu'il a dessinés.

Une fois sèches, les zones colorées s'effacent et l'enfant peut recommencer à dessiner de nouveaux motifs.

On connaît par ailleurs par le document WO 2005/123516 un récipient pour une boisson, ce récipient étant destiné à être rempli une seule fois.

Lorsque le récipient neuf et vide reçoit une boisson froide ou chaude, alors un phénomène de condensation survient à sa surface extérieure et provoque l'humidification d'une encre hydrochrome qui imprègne cette surface extérieure. L'encre hydrochrome devient transparente et révèle des motifs placés juste en dessous. Du fait du caractère irréversible du changement d'état de l'encre, lesdits motifs restent apparents même une fois que le récipient a été vidé. Un utilisateur est donc en mesure de déterminer que ledit récipient a déjà été rempli.

Une telle conception du récipient ne permet de caractériser un remplissage qu'à condition qu'une différence de température significative soit assurée entre les parois du récipient et le liquide qui y est versé pour qu'une condensation puisse se produire sur la surface externe du récipient.

Une telle conception n'est pas adaptée à des récipients destinés à d'autres usages, notamment à recevoir un substrat et une plante en vue de sa culture. En effet, le phénomène de condensation décrit dans le document précité n'est pas engendré dans de tels cas, puisque l'eau éventuellement contenue dans le substrat est à la même température que celle du récipient.

Il n'est par ailleurs pas envisageable de placer l'encre hydrochrome sur la surface intérieure du récipient pour qu'elle soit en contact direct avec l'eau éventuellement contenue dans le récipient. En effet, dans ce cas, un éventuel changement d'état de l'encre hydrochrome ne serait détectable qu'en observant la surface intérieure du récipient. Or, c'est la surface extérieure du récipient qui est destinée à être vue par l'examinateur, la surface intérieure étant masquée par le substrat contenu dans le récipient.

Le document EP 2 269 443 divulgue un récipient comprenant une paroi poreuse intégrant un témoin de présence d'eau. A cet effet, une région de la face externe de ladite paroi poreuse est recouverte d'un graphisme, lui-même recouvert d'une couche d'un matériau sensible à l'humidité qui est opaque en l'absence d'eau et transparent en présence d'eau. Ainsi, le symbole est visible de l'extérieur du récipient lorsque de l'eau est présente dans celui-ci et traverse la paroi poreuse, et invisible en l'absence d'eau dans le récipient. Cependant, compte tenu de sa porosité, la paroi du récipient doit être recouverte, sur sa face externe, d'un matériau étanche à l'eau, sauf à l'emplacement du témoin d'humidité. La fabrication d'un tel récipient est donc complexe.

Le document WO 03/015060 décrit quant à lui un témoin d'humidité à base d'encre hydrofuge. Ce dispositif comprend une étiquette collée sur la face externe de la paroi d'un récipient de sorte à obturer un orifice pratiqué dans ladite paroi. Ladite étiquette est formée successivement d'une couche d'encre hydrofuge, d'une couche de papier, d'une couche adhésive et d'un film transparent. En présence d'eau, l'encre hydrofuge diffuse au travers du papier et devient alors visible de l'extérieur du récipient. Cependant, cette solution nécessite de pratiquer un orifice dans le récipient.

La présente invention a pour but de résoudre ces problèmes en proposant une étiquette comprenant une encre hydrochrome, ladite étiquette pouvant être apposée sur la paroi du récipient de sorte que l'encre hydrochrome soit visible de l'extérieur du récipient, et dont le changement d'état est provoqué par la présence d'humidité directement contenue dans le récipient.

Par « hydrochrome » on entend dans le présent texte une encre présentant un premier aspect à l'état sec et un second aspect différent du premier à l'état mouillé, le premier aspect étant typiquement opaque tandis que le second aspect est transparent. De manière particulièrement avantageuse, un tel changement d'aspect est réversible.

Ainsi, selon un premier aspect de l'invention, celle-ci se rapporte à une étiquette comportant une structure à plusieurs couches qui comprend, dudit volume interne en direction de l'extérieur :
- une première couche d'un matériau poreux, c'est-à-dire perméable à l'eau;
- une deuxième couche colorée non soluble dans l'eau recouvrant au moins une partie de la première couche;
- une troisième couche d'une encre hydrochrome qui recouvre au moins une partie de la deuxième couche colorée, cette encre étant opaque en l'absence d'eau et transparente en présence d'eau.

Cette étiquette est destinée à être apposée sur une paroi intérieure d'un récipient, ladite paroi étant réalisée en un matériau transparent et imperméable à l'eau.

Grâce à cet agencement, de l'eau sous forme quelconque (c'est à dire de l'eau seule ou incorporée à un substrat tel que du terreau) qui est présente dans le récipient traverse la première couche de matériau poreux ainsi que la deuxième couche colorée, de manière à atteindre la troisième couche.

Ce faisant, l'encre hydrochrome change d'état et devient transparente, de sorte que la deuxième couche colorée est révélée visuellement. Une quatrième couche transparente et imperméable protège l'ensemble.

Dans l'ensemble de la présente demande, y compris les revendications, lorsque l'on dit "cette encre étant opaque en l'absence d'eau et transparente en présence d'eau", on sous-entend que ces opacité/transparence sont considérées par rapport à la lumière du domaine visible. Quoique de manière moins avantageuse, une encre qui ne serait pas transparente mais translucide pourrait convenir à l'invention. Cette encre peut présenter une couleur quelconque.

Selon des caractéristiques avantageuses et non limitatives de cette étiquette :
- le matériau poreux est un tissu ;
- ledit tissu comprend des fibres de polyester ;
- ledit matériau poreux est un papier, notamment du papier buvard, du papier filtre ou du carton ;
- ledit matériau poreux est un matériau traité en surface, de sorte qu'il présente des premières régions poreuses et des secondes régions imperméables à l'eau ;
- la seconde couche colorée recouvre toute ou partie de la surface de ladite première couche ;
- ladite seconde couche colorée comporte au moins un motif ;
- l'encre hydrochrome est réversible ;
- l'étiquette comprend, sur la couche d'encre hydrochrome, une couche d'un matériau polymère thermofusible transparent.

Un autre objet de l'invention concerne un récipient, notamment pour la culture de plantes, qui comporte un fond à partir duquel s'élève une paroi périphérique, ce fond et cette paroi définissant ensemble un volume creux dit "volume interne", caractérisé par le fait qu'au moins ladite paroi périphérique comporte une étiquette telle que définie ci-dessus, de telle sorte que ladite paroi périphérique comprenne, dudit volume interne en direction de l'extérieur :
- la première couche de matériau poreux, ladite couche étant en contact avec le volume interne;
- la deuxième couche colorée non soluble dans l'eau ;
- la troisième couche d'encre hydrochrome; et
- une quatrième couche d'un matériau transparent et imperméable à l'eau, formant la paroi du récipient.

De manière particulièrement avantageuse, la quatrième couche est réalisée en un matériau thermoplastique.

Selon un mode de réalisation particulier, le récipient comporte un réceptacle supplémentaire, reçu dans ledit volume interne, et de forme complémentaire de ce dernier, ce réceptacle étant imperméable et pourvu d'au moins une ouverture traversante.

Un autre aspect de l'invention se rapporte à un procédé de fabrication d'un tel récipient.

Ainsi, un premier procédé de fabrication comprend au moins les étapes suivantes :
- fourniture d'une étiquette telle que décrite ci-dessus ;
- mise en place de ladite étiquette dans une cavité de moulage définissant la forme de la quatrième couche ;
- injection d'un matériau thermoplastique dans ladite cavité pour former ledit récipient tridimensionnel.

De manière particulièrement avantageuse, l'étiquette est maintenue dans la cavité de moulage par une charge électrostatique.

Un autre procédé de fabrication comprend au moins les étapes suivantes :
- fourniture d'une étiquette telle que décrite ci-dessus ;
- fourniture d'un récipient formé de la quatrième couche ;
- assemblage de l'étiquette sur la quatrième couche, du côté du volume intérieur.

D'autres caractéristiques et avantages de la présente invention apparaîtront à la lecture de la description qui va suivre de certains modes de réalisation, description qui sera faite en référence aux dessins annexés dans lesquels :
- la figure 1 est une vue en coupe selon un plan transversal et vertical d'un récipient comportant une étiquette conforme à l'invention ;
- la figure 2 est une vue agrandie de la zone de paroi du récipient de la figure 1 délimitée par un cercle ;
- les figures 3, 4, 5 et 6 sont des figures tridimensionnelles destinées à illustrer l'une après l'autre les différentes couches qui forment une étiquette destinée à être intégrée à la paroi du récipient de la figure 1 ;
- les figures 7 et 8 sont des vues analogues à la figure 1 de deux autres variantes de réalisation du récipient ; et
- la figure 9 est vue de dessus du fond du récipient de la figure 8.

Les exemples qui vont être décrits ci-après sont relatifs à un récipient qui a une forme de révolution et, plus précisément, une forme de tronc de cône inversé, ce qui lui confère la forme bien connue des pots de fleurs d'usage courant.

Toutefois, il est à nouveau précisé que le récipient peut avoir une forme et un volume quelconques. Il peut par exemple prendre la forme d'une bouteille, d'un flacon, d'un bol, d'un verre, d'un pot cylindrique, etc.

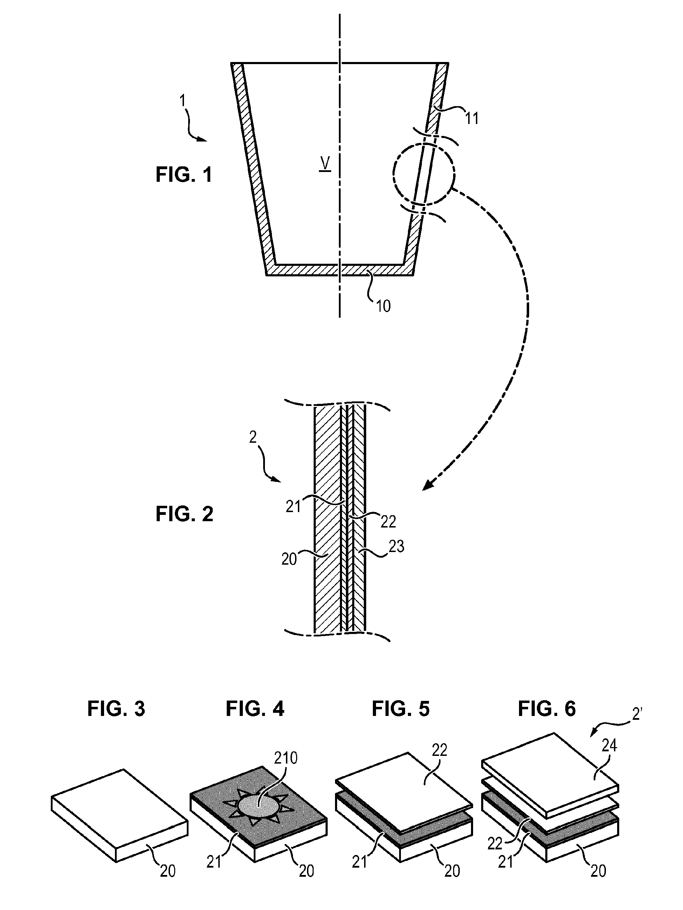

En se reportant à la figure 1, on a représenté le récipient précité en coupe selon un plan transversal et vertical.

Ce récipient référencé 1 comporte un fond 10 qui s'étend dans un plan et à partir duquel s'élève une paroi périphérique 11 divergente au fur et à mesure que l'on s'éloigne du fond 10. Il présente une forme de révolution.

Ce fond 10 et la paroi 11 définissent ensemble un volume creux V autrement appelé "volume interne".

Selon l'invention, au moins la paroi périphérique 11 comprend une structure à plusieurs couches comprenant une étiquette qui sera décrite en détail plus bas. Ladite structure à plusieurs couches peut occuper la totalité de la surface de la paroi périphérique ou seulement une partie de la surface de ladite paroi.

De manière optionnelle, le fond 10, qui est avantageusement d'un seul tenant avec la paroi 11, est également constitué de cette même structure multicouches.

Cette structure est représentée partiellement en coupe transversale à la figure 2.

On précise ici que les épaisseurs des différentes couches qui constituent cette structure ne sont pas représentées à l'échelle, l'objectif étant simplement d'illustrer chacune des couches qui la composent.

Ainsi, cette structure 2 présente, du côté du volume interne V, une première couche 20 d'un matériau poreux, c'est-à-dire perméable à l'eau.

Selon un mode de réalisation, la première couche 20 peut être réalisée en un matériau tel que du papier standard, du papier buvard ou du papier filtre. On peut aussi utiliser du carton.

De manière préférée, la première couche 20 est un tissu. Comme le papier, le tissu est perméable à l'eau.

Par rapport au papier, le tissu présente un certain nombre d'avantages. D'une part, contrairement au papier, le tissu ne se déforme pas lorsqu'il absorbe de l'eau, ce qui limite les risques de délamination de la structure multicouches. Par ailleurs, le tissu peut être nettoyé avec une éponge sans risque même avec une surface abrasive alors que dans le cas du papier les fibres tendent à se détacher du reste de la structure. D'autre part, le tissu est moins sensible au moisissement.

Selon la trame du tissu, une ascension capillaire de l'eau peut également être observée. Plus la trame est resserrée, et plus la vitesse de l'ascension capillaire est réduite. Par ailleurs, la trame ne doit pas être trop resserrée afin de conserver une bonne souplesse du tissu. Il a par ailleurs été constaté que certains tissus polyester présentaient une ascension capillaire du même ordre de grandeur que les papiers les plus performants à cet égard. On choisira donc de tels tissus lorsque l'on souhaite obtenir un effet de capillarité.

Dans tous les cas, on utilisera des épaisseurs et des résistances à l'état sec et humide plus ou moins variables selon l'application envisagée. Selon la porosité et l'épaisseur du matériau employé, l'eau contenue dans le récipient traversera le matériau plus ou moins rapidement. De manière avantageuse, on choisit pour la couche 20 l'épaisseur la plus fine possible pour faciliter sa mise en oeuvre dans une étiquette, notamment pour permettre sa conformation à la paroi du récipient.

La deuxième couche 21 est une couche colorée. Préférentiellement, elle recouvre toute la surface de la première couche, mais cela n'est pas obligatoire. Ainsi, on peut envisager qu'il existe sur la surface extérieure de la première couche une ou plusieurs régions revêtues de cette couche colorée.

Une particularité de cette couche colorée est de ne pas être soluble dans l'eau. D'autre part, ladite couche colorée est perméable à l'eau. De préférence, on utilisera une encre colorée. A cet effet, on pourra utiliser les encres disponibles dans le commerce selon la technique d'impression envisagée, en évitant les encres solubles dans l'eau et les encres imperméables à l'eau.

La troisième couche 22 est une couche d'une encre hydrochrome qui recouvre la deuxième couche colorée, cette encre étant opaque en l'absence d'eau et transparente en présence d'eau. A titre indicatif, l'encre peut par exemple être de couleur blanche lorsqu'elle est opaque. Elle est appliquée sur la deuxième couche 21 selon une épaisseur suffisante pour qu'elle recouvre et masque complètement la couleur de celle-ci lorsqu'elle est opaque.

De manière particulièrement avantageuse, ces trois premières couches 20-22 forment ensemble une étiquette.

Lorsque l'étiquette est destinée à être surmoulée pour former le récipient, l'encre utilisée pour former la couche colorée 21 doit résister à la température de la matière fondue (qui est de l'ordre de 250°C dans le cas du polypropylène, et qui peut être plus ou moins élevée selon le matériau plastique employé) et du moule lors de l'injection. On évite donc dans ce cas d'utiliser une encre graphique de type cire ou xérographie, qui risque de fondre et de se mélanger à l'encre hydrochrome, lui donnant une teinte colorée non désirée. Par ailleurs, l'encre hydrochrome étant légèrement alcaline, il faut également choisir, pour la couche 21, des grades de pigments colorés résistant aux alcalins.

Enfin, la structure comprend une quatrième couche d'un matériau transparent 23 qui est imperméable à l'eau, et qui définit la paroi du récipient proprement dite. Eventuellement, quoique de manière moins avantageuse, ledit matériau peut être translucide. Depuis l'extérieur du récipient, on peut donc observer, au travers de cette quatrième couche 23, l'état de l'encre hydrochrome 22 qui masque ou au contraire laisse apparaître la couche colorée 21. De manière avantageuse mais non obligatoire, cette quatrième couche est réalisée en un matériau thermoplastique. Parmi les matériaux envisageables, on peut citer notamment le polycarbonate, le polyéthylène et le polypropylène. Ladite quatrième couche peut être incolore ou colorée.

Les figures 3 à 6 ne font qu'individualiser les différentes couches dont on vient de parler. A l'exception de la couche 23, ces différentes couches peuvent former un ensemble bidimensionnel 2' tel qu'une étiquette. De préférence, ledit ensemble 2' présente une flexibilité suffisante pour épouser la forme de la paroi du récipient.

Ainsi, la figure 3 représente une portion de la première couche 20, tandis que la figure 4 fait apparaître sur celle-ci la couche colorée 21. On remarquera qu'ici cette couche 21 est bicolore et présente un motif 210 qui a, dans ce mode de réalisation, la forme d'un soleil. Naturellement, on pourra employer tout autre motif ou pluralité de motifs ou utiliser une couche uniformément colorée sans pour autant sortir du cadre de la présente invention.

A la figure 5, on identifie la couche d'encre hydrochrome 22 qui recouvre les couches 20 et 21.

Selon un mode de réalisation, la couche d'encre hydrochrome 22 recouvre la totalité de la couche colorée 21.

De manière alternative, la couche d'encre hydrochrome peut recouvrir uniquement une partie de la couche colorée 21. Par exemple, la couche colorée présente une teinte uniforme et l'encre hydrochrome définit un motif.

Comme illustré sur la figure 6, l'étiquette peut avantageusement comprendre, sur la couche d'encre hydrochrome 22, une couche d'un matériau polymère thermofusible transparent 24, par exemple du polypropylène. Cette couche permet de protéger l'encre hydrochrome tout en préservant sa fonctionnalité. Ladite couche thermofusible 24 permet également d'améliorer les propriétés mécaniques de l'étiquette (notamment sa résistance au déchirement) avant sa mise en oeuvre dans le récipient 1. La couche thermofusible 24 est destinée à venir en contact avec la couche 23 qui forme la paroi du récipient. Ainsi, si le récipient 1 est formé par surmoulage d'un matériau thermoplastique sur l'étiquette, la couche thermofusible 24 fusionne avec le matériau thermoplastique injecté. Dans ce cas, qui est illustré sur la figure 1, l'ensemble 2' formé des couches 20, 21, 22 et le cas échéant 24 est indissociable de la couche 23. Lorsque l'étiquette est destinée à être collée sur un récipient existant, la couche 24 présente avantageusement une face extérieure autocollante recouverte d'un film de protection pelable.

Pour la réalisation de la couche thermofusible 24, différents films de pelliculage en polypropylène (PP) ont été testés. Quel que ce soit le mode d'application dudit film (thermoscellage, adhésif à froid préenduit sur le film ou utilisation d'une colle en spray sur un film nu), les essais de surmoulage ont montré la bonne fusion entre le film de pelliculage et le matériau thermoplastique injecté.

Avant de décrire d'autres modes de réalisation de ce récipient, on se propose ci-dessous d'expliquer les phénomènes qui se produisent lorsque le récipient 1 contient de l'eau.

On peut envisager que le récipient contienne de l'eau proprement dite, mais on peut aussi envisager le cas où le récipient reçoit un substrat humide tel que du terreau.

Quelle que soit l'application envisagée, l'eau contenue dans le récipient 1 traverse petit à petit la première couche poreuse 20, ainsi que la deuxième couche colorée, pour atteindre la troisième couche 22 d'encre hydrochrome. Ce faisant, celle-ci change d'état et devient transparente.

Il convient de noter que, compte tenu de la porosité de la couche 20, le passage de l'eau peut se faire directement dans le sens de l'épaisseur de cette couche mais aussi par capillarité, à partir d'un bord de la couche 20, avec une propagation dans un plan perpendiculaire à l'épaisseur. Ainsi, même si de l'eau n'est présente que dans la partie inférieure du récipient, dès lors que le bord inférieur de ladite couche 20 est au contact de l'eau, l'eau peut imprégner la couche 20 sur une certaine hauteur supérieure au niveau d'eau et ainsi modifier l'aspect de l'encre hydrochrome. Ceci permet donc de révéler, sur une surface importante, même une faible quantité d'eau contenue au fond du récipient. Un tel phénomène est impossible en l'absence d'une telle couche poreuse : en effet, dans un tel cas, seule l'eau présente directement au niveau de l'encre hydrochrome peut modifier l'aspect de l'encre et ainsi être détectée.

Du fait que la quatrième couche 23 est transparente et imperméable cela permet, d'une part, de voir à travers les couches 22 et 23 et éventuellement 24 la présence de la couche colorée 21 et cela permet aussi de constituer une barrière étanche qui empêche l'eau de sortir du récipient. Ceci constitue un premier état du récipient.

Bien entendu, le temps de réponse de l'encre hydrochrome (temps pour passer de l'état opaque à l'état transparent) dépend de la capacité d'absorption du matériau poreux.

Dans le cas de figure où le récipient ne comporte plus d'eau, alors la couche d'encre hydrochrome, qui est préférentiellement réversible, reprend son état initial dans lequel elle est opaque (par exemple la couleur blanche). Dans ces conditions, l'absence d'eau dans le récipient peut être perçue de l'extérieur au travers de la quatrième couche 23, puisque la deuxième couche colorée n'est plus visible. Dans le cas où le récipient est un pot de fleur, un utilisateur comprend qu'il est alors temps de reverser de l'eau dans le récipient.

Comme mentionné plus haut, il est également possible que la couche d'encre hydrochrome 22 ne recouvre qu'une partie de la couche colorée 21. Dans ce cas, la partie de la couche colorée 21 non recouverte de l'encre hydrochrome est visible en permanence depuis l'extérieur du récipient, tandis que la partie recouverte de l'encre hydrochrome est masquée par ladite encre opaque à l'état sec et révélée par ladite encre transparente à l'état humide.

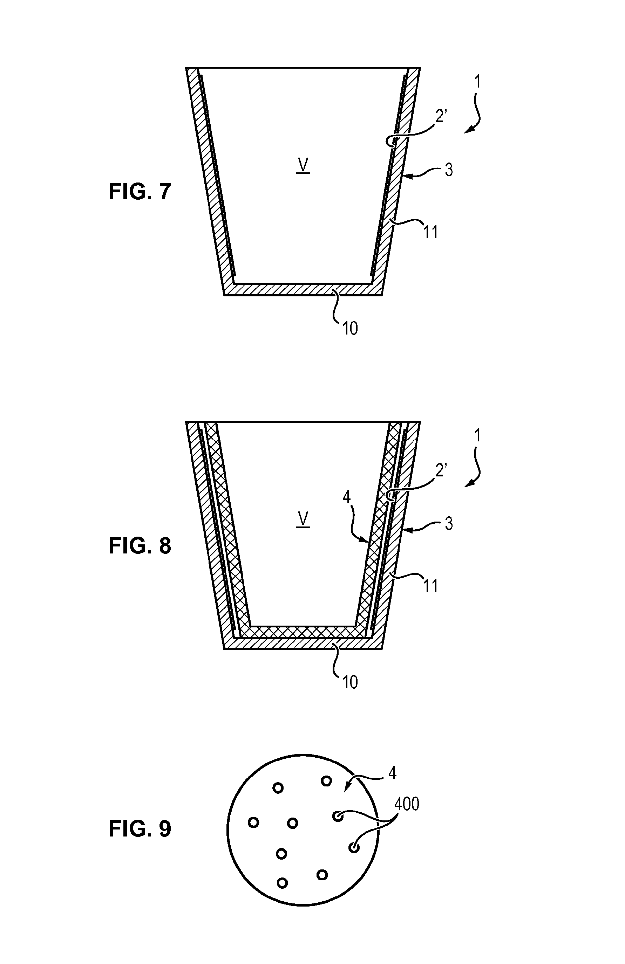

Comme indiqué plus haut, aux figures 7 et 8 sont représentées deux autres formes de réalisation du récipient de la figure 1.

En l'occurrence, lorsque l'on considère le récipient 1 de la figure 7, la quatrième couche 23 forme un récipient 3 comprenant un fond 10 et une paroi périphérique 11 du récipient, l'ensemble unitaire 2' constitué des première, deuxième et troisième couches (et le cas échéant de la couche thermofusible) précitées étant distinct du récipient 3, solidaire de la face intérieure de la couche 23, par exemple collé à la manière d'une étiquette. Ledit ensemble unitaire est typiquement appliqué sur la paroi périphérique, et éventuellement sur le fond du récipient 3.

On a affaire, à la figure 8, au même type de récipient 1 que sur la figure 1 ou la figure 7. Toutefois, afin d'éviter un délitement trop rapide de la première couche 20, on place, dans le volume intérieur V du récipient sur lequel l'ensemble comprenant la couche d'encre hydrochrome est agencé, un réceptacle complémentaire et supplémentaire 4, ce réceptacle étant imperméable. A titre indicatif, il peut s'agir d'un récipient constitué d'une matière plastique opaque.

Cependant, comme le montre particulièrement la figure 9, au moins le fond de ce récipient supplémentaire présente des ouvertures 400 pour le passage de l'eau en direction de la première couche de la structure 2, l'eau se propageant dans le matériau poreux de la couche 20 par capillarité à partir du fond 10 du récipient 1. Dans ce cas, on fait en sorte que la couche 20 de matériau poreux s'étende à partir du fond du récipient, pour que l'eau éventuellement présente au fond du récipient atteigne le bord inférieur dudit matériau poreux.

Par exemple, dans le cas de la culture de plante, ce réceptacle 4 peut être le récipient contenant le substrat et la plante, le récipient 1 remplissant une fonction esthétique et d'indication du degré d'humidité du substrat.

Dans une forme de réalisation avantageuse, le matériau poreux de la première couche 20 est traité en surface de sorte qu'il présente des premières régions poreuses et des secondes régions imperméables à l'eau. Ceci permet d'augmenter considérablement la durée de vie de la structure 2 ainsi constituée et, le cas échéant, d'ajuster les phénomènes de capillarité mentionnés plus haut.

Pour ce faire, on peut utiliser une technique de traitement par plasma de surface ou la technique de chromatogénie, dans le but de rendre le papier localement hydrophobe. Dans le cas où la couche poreuse est suffisamment mince (ce qui est le cas en général du papier mais non du carton), un tel traitement est susceptible de bloquer localement le phénomène de capillarité intervenant dans la couche poreuse 20.

Quel que soit le mode de réalisation du récipient, la technique d'impression de l'encre colorée de la deuxième couche 21 peut être de différentes natures. Ainsi, on peut procéder à une impression par flexographie, héliographie, par la technique offset, par jet d'encre ou sérigraphie.

Cela vaut également pour l'encre hydrochrome. Le dépôt doit homogène.

L'épaisseur de dépôt ne doit pas être trop fine car l'encre ne serait alors pas assez opacifiante à l'état sec, ni trop épaisse car l'encre aurait du mal à être transparente à l'état humide.

Typiquement, l'épaisseur de la couche d'encre hydrochrome est comprise entre 30 et 60 µm.

La sérigraphie semble être la technique de dépôt la mieux adaptée pour le dépôt de l'encre hydrochrome. Des techniques ayant recours à la flexographie nécessitent quant à elles plusieurs passages afin d'obtenir un dépôt suffisamment couvrant à l'état sec.

A titre d'encres hydrochromes, on peut utiliser les encres comprenant des polymères acryliques, de la silice (ou un mélange silice/dioxyde d'aluminium/oxyde de sodium), de l'eau et un solvant (et éventuellement de l'éthylène glycol).

A titre purement indicatif, l'encre "Hydro-Chromic White C-1224" de la société Matsui Shikiso Chemical peut ainsi être utilisée. Il en est de même pour l'encre "C.I.N.O" de la société New Color Chemical.

On décrira maintenant deux procédés de fabrication d'un récipient tel que ceux qui viennent d'être décrits plus haut.

Ainsi, quand on a affaire à un récipient tel que celui de la figure 1, dont la quatrième couche est réalisée en un matériau thermoplastique, alors ce procédé comprend au moins les étapes suivantes :
- fourniture de l'étiquette décrite plus haut, comprenant lesdites première à troisième couches 20 à 22, et le cas échéant la couche thermofusible 24.

On se trouve alors dans la situation de la figure 6 :
- mise en place de cette étiquette dans une cavité de moulage définissant la forme de la quatrième couche 23, la couche 20 se trouvant contre la paroi de la cavité définissant la surface intérieure du récipient,
- injection d'un matériau thermoplastique dans la cavité de moulage pour former ledit récipient tridimensionnel.

Un tel procédé de surmoulage d'une étiquette est connu sous le terme anglo-saxon « In-Mold Labelling » (IML) ; toutefois, à la différence de la présente invention, les étiquettes employées conventionnellement dans ce procédé sont en un matériau plastique non perméable à l'eau, typiquement en polypropylène.

L'étiquette formée des couches 20 à 22 (et le cas échéant 24) devient alors indissociable de la quatrième couche 23.

On notera que l'utilisation d'un tissu comme matériau poreux pour la première couche 20 présente des avantages par rapport au papier. D'une part, afin de pouvoir maintenir l'étiquette sur le noyau (partie mobile du moule), l'étiquette doit être la plus flexible possible afin d'épouser la forme conique du noyau. Dans le cas des papiers, après l'impression de l'ensemble des couches, l'étiquette devient généralement trop rigide pour être maintenue correctement sur le noyau. Malgré l'épaisseur importante des étiquettes à base de tissu (environ 350-450 µm, contre 40-50 µm pour une étiquette standard en polypropylène), celles-ci restent beaucoup plus flexibles que leur équivalent à base de papier. D'autre part, les étiquettes à base de tissu polyester peuvent être chargées électrostatiquement, ce qui n'est pas le cas des étiquettes à base de papier. Ceci permet, comme pour les étiquettes standard à base de polypropylène, de charger électrostatiquement l'étiquette afin de lui permettre d'adhérer au moule.

De manière alternative, un système d'aspiration placé à l'intérieur du noyau peut également être utilisé en vue de maintenir l'étiquette sur ce dernier pendant l'injection du matériau thermoplastique.

Lorsque le récipient est tel que présenté à la figure 7, c'est-à-dire quand la quatrième couche 23 est séparée et forme un ensemble distinct, on préférera alors mettre en oeuvre un procédé qui comprend au moins les étapes suivantes :
- fourniture de l'étiquette comprenant lesdites première à troisième couches 20 à 22, et le cas échéant la couche thermofusible 24 ;
- fourniture d'un récipient 3 formé de la quatrième couche 23 ;
- assemblage de l'étiquette sur la quatrième couche 23 du côté du volume intérieur V, ledit assemblage pouvant par exemple consister en un collage.

Dans ce cas, l'étiquette formée des couches 20 à 22 (et le cas échéant 24), peut éventuellement être ultérieurement retirée et remplacée par une étiquette neuve, par exemple lorsqu'elle a été dégradée.

Sur les figures présentées, l'étiquette couvre une partie substantielle de la surface de la paroi périphérique du récipient.

Cependant, selon les applications visées, il est également possible de n'appliquer l'étiquette (soit par surmoulage IML soit par collage sur un récipient existant) que dans une région déterminée de la paroi du récipient. Par exemple, dans le cas d'un réservoir d'eau, l'étiquette peut être agencée à l'emplacement d'une fenêtre d'indication du niveau. La lecture du niveau d'eau ne se fait donc pas directement en observant l'eau au travers de la fenêtre, ce qui peut être malcommode, mais en observant la couleur de l'étiquette, la partie de l'étiquette en contact avec l'eau présentant un aspect différent de la partie de l'étiquette qui est sèche. En choisissant les aspects à l'état sec et à l'état mouillé suffisamment différents, l'étiquette permet ainsi une lecture plus aisée du niveau d'eau dans le réservoir.

L'invention n'est pas limitée à la culture de plantes mais s'applique de manière plus générale à tout récipient destiné à donner une indication de présence d'eau dans le récipient.

## Revendications

1. Etiquette (2') destinée à être intégrée à la paroi (23) d'un récipient, **caractérisée en ce qu'**elle comprend :
- une première couche (20) d'un matériau poreux, c'est-à-dire perméable à l'eau ;
- une deuxième couche colorée (21) non soluble dans l'eau, recouvrant au moins une partie de la première couche (20) ;
- une troisième couche (22) d'une encre hydrochrome qui recouvre au moins une partie de la deuxième couche colorée, cette encre étant opaque en l'absence d'eau et transparente en présence d'eau.

2. Etiquette selon la revendication 1, **caractérisée en ce que** ledit matériau poreux est un tissu.

3. Etiquette selon la revendication 2, **caractérisée en ce que** ledit tissu comprend des fibres de polyester.

4. Etiquette selon la revendication 1, **caractérisée en ce que** ledit matériau poreux est un papier, notamment du papier buvard, du papier filtre ou du carton.

5. Etiquette selon l'une des revendications 1 à 4, **caractérisée en ce que** ledit matériau poreux est un matériau traité en surface, de sorte qu'il présente des premières régions poreuses et des secondes régions imperméables à l'eau.

6. Etiquette selon l'une des revendications 1 à 5, **caractérisée en ce que** ladite seconde couche (21) colorée comporte au moins un motif (210).

7. Etiquette selon l'une des revendications 1 à 6, **caractérisée par le fait que** ladite encre hydrochrome est réversible.

8. Etiquette selon l'une des revendications 1 à 7, **caractérisée en ce qu'**elle comprend, sur la couche d'encre hydrochrome (22), une couche d'un matériau polymère thermofusible transparent (24).

9. Récipient (1), notamment pour la culture de plantes, qui comporte un fond (10) à partir duquel s'élève une paroi périphérique (11), ce fond (10) et cette paroi (11) définissant ensemble un volume creux dit "volume interne" (V), **caractérisé par le fait qu'**au moins ladite paroi périphérique (11) comporte une étiquette (2') selon l'une des revendications 1 à 8, de telle sorte que ladite paroi périphérique comprenne, dudit volume interne (V) en direction de l'extérieur :
- la première couche (20) de matériau poreux, ladite couche (20) de l'étiquette (2') étant en contact avec le volume intérieur (V);
- la deuxième couche colorée (21) non soluble dans l'eau ;
- la troisième couche (22) d'encre hydrochrome; et
- une quatrième couche (23) d'un matériau transparent et imperméable à l'eau, formant la paroi du récipient.

10. Récipient selon la revendication 9, **caractérisé par le fait que** la quatrième couche (23) est réalisée en un matériau thermoplastique.

11. Récipient selon l'une des revendications 9 ou 10, **caractérisé par le fait qu'**il comporte un réceptacle supplémentaire (4), reçu dans ledit volume interne (V), et de forme complémentaire de ce dernier, ce réceptacle (4) étant imperméable et pourvu d'au moins une ouverture traversante (40).

12. Procédé de fabrication d'un récipient (1) selon la revendication 10, **caractérisé par le fait qu'**il comprend au moins les étapes suivantes :
- fourniture d'une étiquette (2') selon l'une des revendications 1 à 8 ;
- mise en place de ladite étiquette dans une cavité de moulage définissant la forme de la quatrième couche (23) ;
- injection d'un matériau thermoplastique dans ladite cavité pour former ledit récipient tridimensionnel (1).

13. Procédé selon la revendication 12, **caractérisé en ce que** l'étiquette est maintenue dans la cavité de moulage par une charge électrostatique.

14. Procédé de fabrication d'un récipient (1) selon la revendication 10, **caractérisé par le fait qu'**il comprend au moins les étapes suivantes :
- fourniture d'une étiquette (2') selon l'une des revendications 1 à 8 ;
- fourniture d'un récipient (3) formé de la quatrième couche (23) ;
- assemblage de l'étiquette sur la quatrième couche (23), du côté du volume intérieur (V).
